# EUROPÄISCHE PATENTSCHRIFT

(11) **EP 0 912 148 B1**
(45) Veröffentlichungstag und Bekanntmachung des Hinweises auf die Patenterteilung: **28.12.2005**
(21) Anmeldenummer: 98910587.9
(22) Anmeldetag: 06.02.1998
(51) Int. Cl.: A61F 5/058

(54) **ORTHOPÄDISCHE SCHIENE**
ORTHOPEDIC SPLINT
ATTELLE ORTHOPEDIQUE

(30) Priorität: 08.02.1997 DE 29702186 U
(43) Veröffentlichungstag der Anmeldung: 06.05.1999
(73) Patentinhaber: Medi Bayreuth Weihermüller & Voigtmann GmbH & Co. KG, 95448 Nürnberg (DE)
(72) Erfinder: STROBEL, Michael, D-94360 Mitterfels (DE); EICHHORN, Jürgen, D-94360 Mitterfels (DE); HAUSLADEN, Karl, D-94315 Straubing (DE)
(74) Vertreter: Schuhmann, Albrecht
(86) Internationale Anmeldenummer: PCT/DE1998/000328
(87) Internationale Veröffentlichungsnummer: WO 1998/034571

(56) Entgegenhaltungen:
- EP-A- 0 067 319
- EP-B- 0 489 790
- DE-A- 4 411 469
- DE-A- 4 412 765
- US-A- 3 831 467
- US-A- 5 387 185
- Duden Fremdwörterbuch, 3. Auflage, 1974, S.544.
- Baehler André R.: Orthopädie Technische Indikationen; Verlag Haus Huber; S.424, S.429-431.
- Hohmann; Uhlig; Orthopädische Technik; 8. Auflage; 1990; Ferdinand Enke Verlag; Stuttgart; S.347-351.

## Beschreibung

Die Erfindung betrifft eine orthopädische Schiene für die Ruhigstellung des Knies, insbesondere nach Eingriffen am hinteren Kreuzband.

Eine orthopädische Schiene für die Ruhigstellung des Knies, bestehend aus einem Umschlagteil mit Stabilisierungsstäben, die in Taschen angeordnet sind, sowie Klettbändern für die Fixierung des um ein Bein geschlagenen Umschlagteils, ist beispielsweise unter dem Produktnamen medicom Classic bekannt. Das Umschlagteil weist Flügel auf, die mittels Klettbändern an ihm fixiert sind, und durch die die Schiene an unterschiedliche Beingrößen angepaßt werden kann. Diese Schiene wird bei Verletzungen des vorderen Kreuzbandes, des Meniskusses und dergleichen eingesetzt. Das hintere Kreuzband steht bei normaler Streckstellung des Knies unter Spannung. Beim Liegen zieht das Eigengewicht des Beines den Unterschenkel in die sogenannte hintere Schublade und verstärkt so die Spannung auf das hintere Kreuzband. Die daraus resultierende Belastung des hinteren Kreuzbandes, insbesondere nach einer operativen Rekonstruktion, soll möglichst vermieden werden, um die Einheilung zu beschleunigen und einer Lockerung des Bandes vorzubeugen.

Aufgabe der vorliegenden Erfindung ist es daher, eine orthopädische Schiene zur Ruhigstellung des Knies zu schaffen, mittels der das hintere Kreuzband während des Heilungsprozesses entlastet wird.

Diese Aufgabe wird mit den im kennzeichnenden Teil des Anspruchs 1 genannten Merkmalen gelöst. Fortbildungen und vorteilhafte Ausführungen der Erfindung sind in den weiteren Ansprüchen umfaßt.

Erfindungsgemäß weist eine orthopädische Schiene der eingangs genannten Art, bestehend aus einem Umschlagteil mit Stabilisierungsstäben, sowie Bändern für die Fixierung des um ein Bein geschlagenen Umschlagteils, im Wadenbereich eine Wadenpelotte auf. Vorzugsweise überragt die Wadenpelotte die Schiene in Richtung auf die Achillessehne und weist an ihrem unteren Ende einen Einschnitt auf und umfaßt dort die Achillessehne auf beiden Seiten.

Nach einer bevorzugten Ausführung der Erfindung besteht die Wadenpelotte aus einem Schaumstoffkörper und ist auf ihrer dem Bein abgewandten Seite mit einer Kunststoffschale verstärkt, wobei der Schaumstoffkörper einen Radius für das Anliegen an der Wade aufweist. Die Wadenpelotte ist mittels eines Klettverschlusses auf der Innenseite des Umschlagteils befestigt.

Im folgenden wird die Erfindung anhand von Zeichnungen beispielhaft näher beschrieben. Dabei zeigen:
- Fig. 1: eine Draufsicht auf eine orthopädische Schiene mit Wadenpelotte in aufgeschlagenem Zustand;
- Fig. 2: eine schematische Darstellung der Schiene von Fig. 1 im angelegten Zustand;
- Fig. 3: eine aufgeschlagene, gegenüber der Fig. 1 leicht abgewandelte, Schiene ohne eingesetzte Wadenpelotte;
- Fig. 4: eine Draufsicht auf eine Wadenpelotte;
- Fig. 5: eine Seitenansicht zu Fig. 4;
- Fig. 6: einen Schnitt entlang der Linie A-A von Fig. 4;
- Fig. 7: einen Schnitt entlang der Linie B-B von Fig. 4.

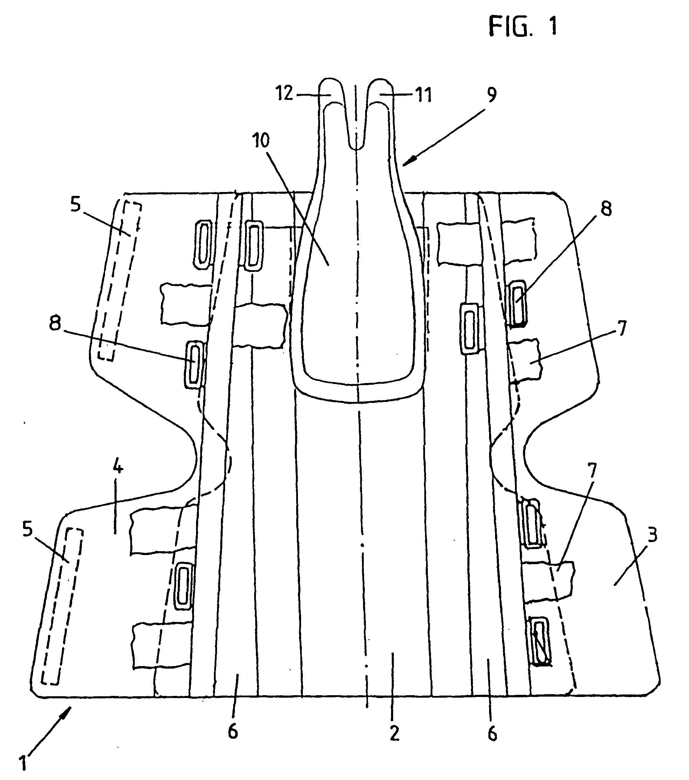

Die orthopädische Schiene 1 zur Ruhigstellung des Knies gemäß Fig. 1 besteht aus einem flexiblen, vorzugsweise textilen Umschlagteil mit einem Mittelteil 2 und zwei Seitenteilen 3, 4. Das Mittelteil 2 und die Seitenteile 3, 4 sind, beispielsweise über Klettverschlüsse, miteinander lösbar verbunden, um verschiedenen Beingrößen, bzw. -zuständen angepaßt zu werden. An den Seiten, d.h. den Bereichen, die bei angelegter Schiene seitlich am Bein liegen, sind längs Stabilisierungsstäbe 6 angeordnet, die vorzugsweise herausnehmbar in Taschen angeordnet sind. Das Umschlagteil ist außen mit einem Klettflausch versehen, mit dem Klettbänder 5 in Eingriff bringbar sind, die außen in dem Seitenteil 4 angeordnet sind. Für den festen Sitz am Bein sorgen radiale Klettbänder 7, die innen am Umschlagteil befestigt sind und durch gegenüberliegende Ösen 8 nach außen geführt und umgelegt werden, wo sie ebenfalls mit dem Klettflausch verbindbar sind. Am unteren Ende des Umschlagteils ist eine Wadenpelotte 9 angeordnet, die einen bauchigen oberen Abschnitt 10 aufweist, der sich nach unten verjüngt und in zwei Enden 11, 12 ausläuft, die einen Spalt zwischen sich aufweisen. Die Wadenpelotte 9 überragt unten das Umschlagteil.

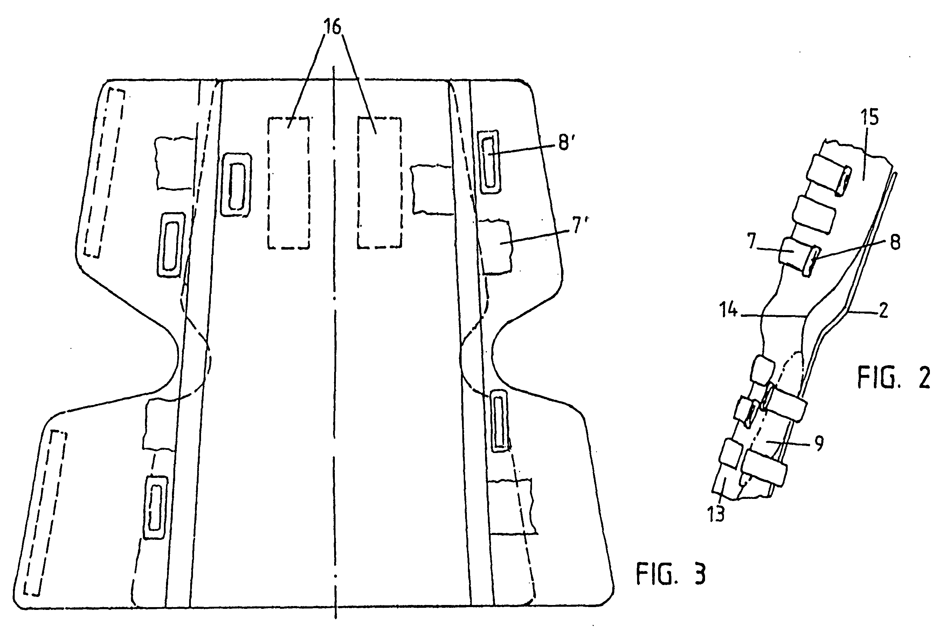

In Fig. 2 ist schematisch dargestellt, wie die Schiene 1 am Bein angelegt ist. Zu erkennen ist ein Bein mit Oberschenkel 15, Knie 14 und Unterschenkel 13. Die Schiene ist mit durch die Ösen 8 geführten Klettbändern fest um das Bein geschlungen. Die oberen, vorne liegenden Klettbänder drücken dabei den Oberschenkel nach hinten, die unteren, hinteren Klettbänder dienen zur Unterstützung der Wadenpelotte. Die Wadenpelotte 9, die mit dem Mittelteil 2 des Umschlagteils mittels eines Klettverschlusses verbunden ist, drückt auf die Wade und wirkt als nach vorne gerichtete Kraft auf den Unterschenkel 13. Dadurch wird verhindert, daß der Unterschenkel im Kniebereich in die sogenannte "hintere Schublade" drängt, was zu einem lockeren Einwachsen des hinteren Kreuzbandes nach einem Eingriff führen würde. Zu erkennen ist auch, daß sich die Wadenpelotte bis in den Achillessehnenbereich erstreckt, den sie mit ihren Enden 11, 12 seitlich stützt.

Fig. 3 stellt eine etwas abgewandelte, kleinere Ausführung der Schiene von Fig. 1 dar, die weniger Klettbänder 7' und Ösen 8' aufweist. Die Wadenpelotte ist hier nicht eingesetzt und die Flauschbänder 16, die mit Klettbändern der Wadenpelotte in Eingriff bringbar sind, sind zu erkennen. Der Klettverschluß zwischen der Wadenpelotte und dem Umschlagteil erlaubt eine individuelle Einstellung am Bein.

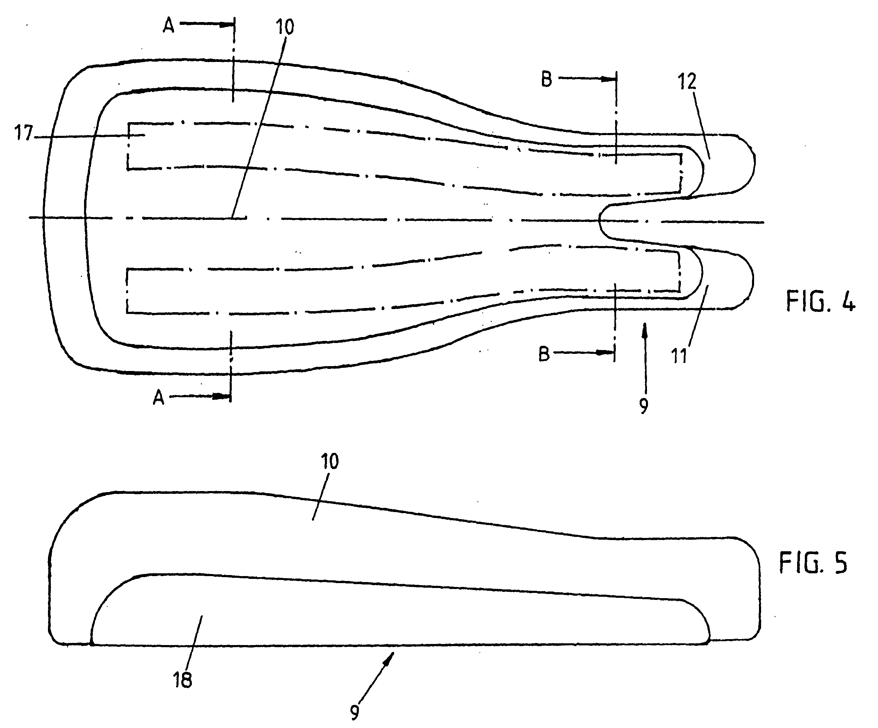

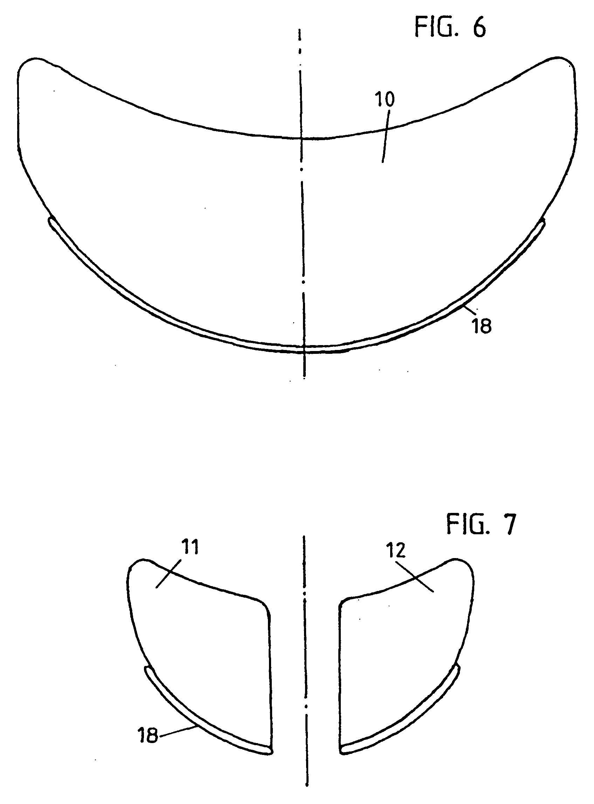

Die Wadenpelotte 9 gemäß den Fig. 4 - 7 weist einen bauchigen oberen Abschnitt 10 auf, der, wie in Fig. 1 bereits dargestellt, sich nach unten verjüngt und in zwei Enden 11, 12 ausläuft. Sie besteht aus einem an der Wade anliegenden Schaumstoffkörper, der innen mit einem hautfreundlichen Material überzogen ist und außen von einer Kunststoffschale 18 abgestützt wird. Die Kunststoffschale erstreckt sich im über den größeren Teil der Länge der Wadenpelotte 9 einschließlich ihrer Enden 11, 12. Die Wadenpelotte 9 weist innen und außen jeweils einen an die Wade angepaßten radialen Radius auf. In Fig. 4 sind die Klettbänder 17 angedeutet, die außen auf der Kunststoffschale 18 angeordnet sind und mit den Flauschbändern 16 von Fig. 3 in Eingriff bringbar sind.

## Patentansprüche

1. Orthopädische Schiene (1) für die Ruhigstellung des Knies, insbesondere nach Eingriffen am hinteren Kreuzband, bestehend aus einem Umschlagteil (3,4) mit Stabilisierungsstäben (6), sowie Bändern für die Fixierung des um ein Bein geschlagenen Umschlagteils,
**dadurch gekennzeichnet,**
**dass** das Umschlagteil im Wadenbereich eine auf die Wade drückende und als nach vorne gerichtete Kraft auf den Unterschenkel wirkende Wadenpelotte (9) aufweist.

2. Orthopädische Schiene nach Anspruch 1,
**dadurch gekennzeichnet,**
**daß** die Wadenpelotte (9) die Schiene (1) in Richtung auf die Achillessehne überragt.

3. Orthopädische Schiene nach Anspruch 2,
**dadurch gekennzeichnet,**
**daß** die Wadenpelotte (9) an ihrem unteren Ende einen Einschnitt aufweist und dort die Achillessehne auf beiden Seiten umfaßt.

4. Orthopädische Schiene nach einem der vorhergehenden Ansprüche,
**dadurch gekennzeichnet,**
**daß** die Wadenpelotte (9) aus einem Schaumstoffkörper besteht und auf ihrer dem Bein abgewandten Seite mit einer Kunststoffschale (18) verstärkt ist,
und **daß** der Schaumstoffkörper einen Radius für das Anliegen an der Wade aufweist.

5. Orthopädische Schiene nach einem der vorhergehenden Ansprüche,
**dadurch gekennzeichnet,**
**daß** die Wadenpelotte (9) mittels eines Klettverschlusses auf der Innenseite des Umschlagteils befestigt ist.

6. Orthopädische Schiene nach einem der vorhergehenden Ansprüche,
**dadurch gekennzeichnet,**
**daß** das Umschlagteil aus miteinander in verschiedenen Positionen verbindbaren Einzelteilen (2, 3, 4) besteht.

## Claims

1. Orthopedic splint (1) for immobilizing the knee; particularly after surgery of the posterior cruciate ligament, consisting of a covering part (3, 4) with stabilizing rods (6), and straps for fixing the covering part wrapped round a leg, **characterized in that**, in the calf area, the covering part has a calf padding (9) which applies pressure to the calf and which acts as a forwardly directed force on the lower leg.

2. Orthopedic splint according to Claim 1, **characterized in that** the calf padding (9) protrudes beyond the splint (1) in the direction of the Achilles tendon.

3. Orthopedic splint according to Claim 2, **characterized in that** the calf padding (9) has an incision at its lower end and there encloses the Achilles tendon on both sides.

4. Orthopedic splint according to one of the preceding claims, **characterized in that** the calf padding (9) consists of a foam body and is reinforced with a plastic shell (18) on its side facing away from the leg, and **in that** the foam body has a radius for bearing on the calf.

5. Orthopedic splint according to one of the preceding claims, **characterized in that** the calf padding (9) is secured on the inner side of the covering part by means of a touch-and-close fastener.

6. Orthopedic splint according to one of the preceding claims, **characterized in that** the covering part consists of individual parts (2, 3, 4) which can be connected to one another in different positions.

## Revendications

1. Attelle orthopédique (1) destinée à immobiliser le genou, en particulier après intervention chirurgicale au niveau du ligament croisé arrière, ladite attelle orthopédique étant constituée d'une partie d'enveloppe (3, 4) comportant des tiges de stabilisation (6) ainsi que des bandes de fixation de la partie d'enveloppe enveloppant une jambe,
**caractérisée en ce que** la partie d'enveloppe comporte dans la région du mollet une pelote de mollet (9) pressant sur le mollet et exerçant une force dirigée vers l'avant sur la jambe.

2. Attelle orthopédique selon la revendication 1, **caractérisée en ce que** la pelote de mollet (9) dépasse de l'attelle (1) en direction du tendon d'Achille.

3. Attelle orthopédique selon la revendication 2, **caractérisée en ce que** la pelote de mollet (9) présente à son extrémité inférieure une entaille au niveau de laquelle elle entoure le tendon d'Achille des deux côtés.

4. Attelle orthopédique selon l'une des revendications précédentes, **caractérisée en ce que** la pelote de mollet (9) est constituée d'un corps de matière alvéolaire et est renforcée du côté opposé à la jambe par une coque de matière plastique (18), et **en ce que** le corps de matière alvéolaire a un rayon permettant l'application sur le mollet.

5. Attelle orthopédique selon l'une des revendications précédentes, **caractérisée en ce que** la pelote de mollet (9) est fixée à l'aide d'une bande "Velcro" du côté intérieur de la partie d'enveloppe.

6. Attelle orthopédique selon l'une des revendications précédentes, **caractérisée en ce que** la partie d'enveloppe est constituée de parties individuelles (2, 3, 4) pouvant être reliées en différentes positions.
